# EUROPEAN PATENT SPECIFICATION

(11) **EP 2 519 261 B1**
(45) Date of publication and mention of the grant of the patent: **27.11.2013**
(21) Application number: 10798345.4
(22) Date of filing: 27.12.2010
(51) Int. Cl.: A61K 47/10, A61K 9/00, A61K 31/44

(54) **Oral liquid pharmaceutical composition of nifedipine**
Orale flüssige pharmazeutische Zusammensetzung aus Nifedipin
Composition pharmaceutique liquide de nifédipine à prise par voie orale

(30) Priority: 28.12.2009 EP 09382308
(43) Date of publication of application: 07.11.2012
(73) Proprietor: Laboratorio Reig Jofre S.A., 08970 Sant Joan Despí (ES)
(72) Inventor: REIG LÓPEZ, Isabel, E-08970 Sant Joan Despí (ES); NAVARRO PUJOL, Francesc, E-08970 Sant Joan Despí (ES); BOIX MONTAÑES, Antonio de Padua, E-08970 Sant Joan Despí (ES); NIETO ABAD, Carlos, E-08970 Sant Joan Despí (ES); ALCALDE AGUILAR, Pilar, E-08970 Sant Joan Despí (ES); DOMENECH BERROZPE, José, E-08970 Sant Joan Despí (ES); BORRÁS SCHIERLOH, José María, E-08970 Sant Joan Despí (ES)
(74) Representative: ZBM Patents
(86) International application number: PCT/EP2010/070749
(87) International publication number: WO 2011/080246

(56) References cited:
- US-A- 4 537 898
- US-A- 4 857 312
- US-A1- 2009 130 198
- HELIN-TANNINEN, M. ET AL: "Enteral suspension of nifedipine for neonates. Part 1. Formulation of nifedipine suspension for hospital use", JOURNAL OF CLINICAL PHARMACY AND THERAPEUTICS., vol. 26, 2001, pages 49-57, XP002577245, BLACKWELL SCIENTIFIC PUBLICATION, OXFORD. ISSN: 0269-4727 cited in the application

## Description

The present invention refers to the field of pharmacy. More specifically, the invention relates to the development of new pharmaceutical compositions.

### BACKGROUND ART

Nifedipine is a known dihydropyridine drug which acts as a blocking agent of the cell calcium channel. The corresponding chemical name is 4-(2'-nitrophenyl)-2,6-dimethyl-3,5-dicarbomethoxy-1,4-dihydropyridine and it is represented by the formula:

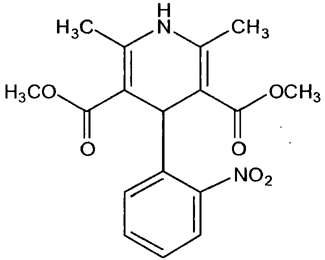

Nifedipine inhibits the contractile process of the vascular smooth muscle leading to arterial dilatation which encompasses an increase of the blood flow and the myocardial oxygenation. Nifedipine acts less remarkably over the cardiac muscle reducing its contraction potential and cardiac conductivity, and therefore also contributing to the increase of the blood flow.

Nifedipine is marketed by Bayer under the trademark of Adalat^{®} in the pharmaceutical form of tablets, soft and hard capsules, with and without modified release profiles, and doses of 10, 20, 30, or 60 mg. Adalat^{®} is approved in the European Union (EU) and the United States of America as an antihypertensive, antianginal agent, and as a dilatator of the peripheral vascular system. In the EU, nifedipine is indicated for the treatment of arterial hypertension, angina and Reynaud's phenomenon.

Several off-label uses have been described for nifedipine, for example as a tocolytic agent, i.e. as an inhibitor of preterm labour. Nifedipine is preferred to traditional beta-agonists for the treatment of threatened preterm labour because of the lower side-effects of nifedipine (cfr. King JF. et al. Cochrane Database of Systematic Reviews 2003, Issue 1. Art. No.: CD002255). The studies reviewed by King et al. used high dosages regimes to stop pre-term contractions ranging from 30 mg/day to 160 mg/day, and dosage regimes of 40-120 mg/day for maintenance treatments lasting for several weeks. King et al. complain about not having at disposal a suitable pharmaceutical form of nifedipine for the administration in such treatments.

The marketed oral soft gelatine capsules of Adalat^{®} for immediate release of nifedipine comprise such amount of PEG that they cannot be used in cases where a high dose of nifedipine is required, since it would lead to a daily intake of PEG above that considered acceptable by the World Health Organization (cfr. Who Technical Report 1980, vol. 648, pg. 19). Further, Adalat compositions do not allow adjusting doses for the requirements of each individual and of the off-label uses of nifedipine.

US 2009/0130198A1 discloses oral compositions comprising surfactants forming an emulsion when are in contact with the gastrointestinal fluids. To delivery the composition to the gastrointestinal tract the disclosed composition is in the form of a sealed soft or hard capsule.

Clinical and officinal oral liquid compositions of nifedipine have been developed to overcome dosage and administration limitations of solid oral compositions. Liquid oral compositions of nifedipine have showed to be the most biopharmaceutical efficient form of the drug.

However, nifedipine is a very insoluble product which requires one or more organic co-solvents or solubilizing agents to get a true dissolution. US 4,537,898 discloses oral liquid compositions of nifedipine comprising polyethyleneglycol (PEG) and derivatives thereof, and optionally its mixtures with other diluents, for example water, ethanol and glycerol. Similarly, US 4,857,312 discloses nifedipine solutions which are suitable for spraying comprising PEG, ethanol and polyvinylpyrrolidone. Unfortunately, the requirement of the use of PEG and PEG derivatives makes the above referred compositions unsuitable due to toxicological limitations for their use when a high daily dose of nifedipine is required. No data on bioavailability and inter-individual variability of compositions comprising nifedipine is provided.

Still further, the unpleasant taste of such solutions usually impairs the treatment compliance.

Where no officinal compositions of nifedipine are available, the liquid composition for hospital use with the specific dose required for each individual is obtained by the preparation of extemporaneous suspensions from bulk nifedipine or immediate release tablets (cf. Helin-Tanninen, M. J. Clin. Pharm. Therap.; 2001, 26, 49-57). These compositions are known to be impaired by the poor dissolution rate of nifedipine which decreases its absorption and impairs the reproducibility of the compositions and thus the variability of its efficacy.

McGilveray et al., Phar. Res., 1992, vol. 9(5), pgs. 683-686, discloses the effect of an acute dose of alcohol on the bioavailability of Adalat^{®}. The results of the study showed that the concomitant administration of a solid oral Adalat^{®} and an acute dose of 75 ml of ethanol increases the bioavailability of nifedipine mainly due to the inhibition effect of ethanol over the metabolism of nifedipine. The results of the co-administration regime proposed shown a high inter-individual variability on the systemic absorption of nifedipine. Further, the high amount of ethanol administered by McGilveray et al. makes the administration regime proposed therein to be absolutely unsuitable for pharmaceutical use due to the toxic effects of such high ethanol doses. The administration of little amounts of ethanol is preferred for pharmaceutical products.

Further, a great variability of nifedipine serum levels have been described in the literature when Adalat^{®} compositions are administered, even in the sustained release form (cf. Marin, T. Z. et al, Journal of Obstetrics and Gynaecology, 2007, 27 vol.3, pgs. 260-263).

Additionally, officinal compositions can not assure as high reproducibility as an industrial process.

Therefore, as extracted from the state of the art exposed above, it would be desirable to have at one's disposal improved pharmaceutical compositions of nifedipine allowing the administration, in one or more intakes, of the daily dose of nifedipine which is required for the off-labelled uses of nifedipine, for example to stop pre-term labour, without any toxicological risk due to an overdose of PEG or PEG-ylated excipients.

### SUMMARY OF THE INVENTION

The applicant has found that an oral pharmaceutical composition comprising 0.1%-1% of nifedipine, 38%- 58% of ethanol, 4%-12% of water, and glycerine in an amount comprised between 30% and 50%, allows the administration, in one or more intakes, of the daily dose of nifedipine which is required for the off-labelled uses of nifedipine, for example to stop pre-term labour which may exceed 120 mg of nifedipine, without any toxicological risk due to an overdose of PEG or PEG-ylated excipients.

Surprisingly, the pharmaceutical composition of the invention further allows decreasing the inter-individual variability on the systemic absorption of nifedipine when administered in the form of Adalat alone or when an acute dose of ethanol and Adalat are co-administered, while increasing bioavailability in respect of Adalat. This is advantageous since it allows to obtain reproducible physiological effects on individuals and to design the most suitable administration routines for the authorized indications and the off-labelled uses of nifedipine.

Although each one of above advantages of the pharmaceutical composition of the invention is a major development in the art, the combination of both renders a pharmaceutical composition especially suitable for some of the off-labeled uses of nifedipine requiring a very controlled drug effect, i.e. decreased inter-individual variability, and the administration of high doses of nifedipine.

The improved effect of the pharmaceutical compositions of the invention is achieved without requiring the use of PEG or PEG-ylated excipients and with a content of ethanol which makes the composition of the present invention suitable for pharmaceutical use, and allows the use of a higher amount of inert diluents such as water and glycerine. In addition, the pharmaceutical compositions of the present invention exhibit a minimum toxicological risk and provide improved taste. Therefore, it is advantageous since it allows having at one's disposal a pharmaceutical composition of nifedipine which allows being readily prepared in flexible doses to match the different clinical and officinal needs, while using less toxic solvents and improving the taste of PEG based compositions

Moreover, the pharmaceutical compositions of the present invention show a suitable shelf-stability, meeting the requirements for industrial preparation processes and marketing, assuring reproducibility on the preparation of the pharmaceutical compositions and its commercialization.

Thus, as a first aspect, the invention provides an oral liquid pharmaceutical composition which is a solution comprising: a) nifedipine in an amount comprised between 0.1% and 1%; b) ethanol in an amount comprised between 38% and 58%; c) water in an amount comprised between 4% and 12%; d) glycerine in an amount comprised between 30% and 50%; and e) optionally, other pharmaceutical excipients or carriers; the sum total of components being 100%, provided that the composition is substantially free of polyethyleneglycol and derivatives thereof according to claim 1. As shown in the Examples provided herein, the pharmaceutical composition of the invention surprisingly improves bioavailability of nifedipine and reduces the dispersion of the inter-individual absorption of the drug, even compared with the results provided by McGilveray et al. when co-administering Adalat^{®} and a much higher amount of ethanol. The reduced inter-individual variability of the absorption of nifedipine also reduces toxicity risks and inefficacy thanks to the more predictable behaviour of the pharmaceutical composition. Furthermore, the reduced amount of ethanol of the pharmaceutical composition of the present invention makes it suitable for medical and pharmacy use.

The decrease of the inter-individual variability of the absorption of nifedipine, its improved bioavailability and the low toxicological risk makes the pharmaceutical composition of the invention suitable for its use in the treatment of acute conditions wherein high doses of nifedipine and rapid high plasmatic concentrations are required and reduced inter-individual variability of the drug effect are desired, for example to stop preterm labour or in case of acute angina or stroke. Thus, a second aspect of the invention relates to the pharmaceutical composition of the present invention for use as a medicament. The pharmaceutical composition of the invention can be used for stopping and/or treating acute conditions, preferably preterm labour, angina, stroke, or Raynaud Syndrome. In a preferred embodiment, the pharmaceutical composition is used for stopping preterm labour.

### DETAILED DESCRIPTION OF THE INVENTION

In the present invention, unless explicitly stated, all percentages are given in a weight basis with regard to the total weight of the unitary liquid pharmaceutical composition.

As used in the present invention, the term "substantially free" refers to a composition which does not include a component at all or can include it in a residual amount, i.e up to 0.1 % in weight, more particularly up to 0.01%.

Polyethyleneglycol and their derivatives are known as solubilizing agents and diluents. The pharmaceutical composition of the invention is substantially free of polyethyleneglycol. Preferably, the composition of the invention is free of polyethyleneglycol. In a preferred embodiment of the first aspect of the invention, the composition is substantially free of polyethyleneglycol and derivatives thereof. More preferably, the composition of the invention is free of polyethyleneglycol and derivatives thereof.

As used in the present invention, the term "derivatives of polyethylene glycol" refers to compounds which are structurally related to polyethylene glycol comprising a polyethylene base structure, which can be modified by the introduction of different chemical groups. Concretely, the derivatives of polyethylene glycol in the context of the present invention are polyethylene glycol alkyl ethers, polyethylene glycol fatty acid esters, polyethylene glycol sorbitan fatty acid esters, or polyethylene glycol oxystearates. Specifically examples of PEG derivatives are polyethylene glycol (20)-sorbitan monolaurate, polyethylene glycol (4)-sorbitan monolaurate, polyethylene glycol (20)-sorbitan monopalmitate, polyethylene glycol (20)-sorbitan tristearate, polyethylene glycol (20)-sorbitan monooleate, polyethylene glycol (20)-sorbitan trioleate, and polyethylene glycol oxystearate oxyethylated with ethylene oxide.

Thus, the composition of the invention is substantially free, or totally free, of PEG and PEG derivatives. The PEG derivatives are selected from the group consisting of polyethylene glycol (20)-sorbitan monolaurate, polyethylene glycol (4)-sorbitan monolaurate, polyethylene glycol (20)-sorbitan monopalmitate, polyethylene glycol (20)-sorbitan tristearate, polyethylene glycol (20)-sorbitan monooleate, polyethylene glycol (20)-sorbitan trioleate, and polyethylene glycol oxystearate oxyethylated with ethylene oxide.

In a particular embodiment of the first aspect of the invention, the pharmaceutical composition of the invention is substantially free of any other solubilizing agent or diluent. More particularly, the composition is free of any other solubilizing agent or diluent. All the preferred and particular embodiments of the invention enclosed in this detailed description are also preferred embodiments of this particular embodiment wherein the composition is free, or substantially free, of any other solubilizing agent or diluent.

In the present invention, the term "solution" refers to a clear liquid pharmaceutical composition intended for oral use wherein the pharmaceutical product, i.e. nifedipine, and the excipients are substantially dissolved in a solvent system.

In a preferred embodiment, the amount of glycerine in the oral liquid pharmaceutical compositions of the invention is comprised between 40% and 46%, more preferably between 42% and 44%.

The amount of ethanol in the pharmaceutical composition of the invention is comprised between 38% and 58%. Preferably, the amount of ethanol is comprised between 43% and 53%. More preferably, the amount of ethanol is comprised between 45% and 50%.

The amount of water in the pharmaceutical composition of the invention is comprised between 4% and 12%. Preferably, the amount of water is comprised between 6% and 10%. More preferably, the amount of water is comprised between 7% and 9%.

In a particularly preferred embodiment, the oral liquid pharmaceutical composition according to the first aspect of the present invention has the following composition: a) nifedipine in an amount comprised between 0.1% and 1.0%; b) ethanol in an amount comprised between 43% and 53%; c) water in an amount comprised between 6% and 10%; and d) glycerine in an amount comprised between 40% and 46%.

In a still more preferred embodiment, the oral liquid pharmaceutical composition according the first aspect of the present invention is a solution having the following composition: a) nifedipine in an amount comprised between 0.3% and 0.7%; b) ethanol in an amount comprised between 45% and 50%; c) water in an amount comprised between 7% and 9%; d) glycerine in an amount comprised between 42% and 44%. More preferably the composition further comprises e) at least one preservative in an amount comprised between 0.05% and 0.5%; f) at least one sweetener in an amount comprised between 0.02% and 0.8%; and g) optionally at least one colouring or flavouring agents, each of them in an amount comprised between 0.01 % and 0.07%.

In addition, the pharmaceutical compositions of the invention can also comprise pharmaceutically acceptable preservatives, sweeteners, colouring and flavouring agents.

Suitable preservatives for oral liquid pharmaceutical compositions can be selected from phenoxyethanol, imidurea, methylparaben, ehylparaben, propylparaben, butylparaben and its salts, and mixtures thereof. Preferred preservatives are ethyl and methylparaben salts and mixtures thereof.

Suitable sweeteners for oral liquid pharmaceutical compositions can be, for example, maltitol, aspartame, erythritol, lactitol, fructose, sucrose, alitame, hesperedine, neohesperedin dihydrochalcone, cyclamate salts, saccharin or acesulfame.

Further, the pharmaceutical compositions of the invention can comprise flavouring and colouring agents selected from those known by the person skilled in the art, in order to improve the taste and appearance of the product.

Other conventional excipients may be employed in the pharmaceutical compositions of the present invention, for example, buffering systems, viscosity enhancing agents, additional sweeteners, and their mixtures.

Generally, doses of the oral liquid pharmaceutical compositions of the invention range from 3 to 200 mg of nifedipine per unitary dose, preferably 5.5 - 30 mg.

Generally, liquid pharmaceutical compositions according to the invention comprise a concentration of nifedipine from 1 to 10 mg/ml, more preferably from 3 to 7 mg/ml.

It also forms part of the invention a process for the preparation of a pharmaceutical composition as defined above.

As an example, a pharmaceutical solution according to the invention can be prepared by mixing an ethanol aqueous solution comprising an amount of ethanol comprised between 43% and 53% of the total composition, which may comprise sweeteners, preservatives or flavouring agents, first with nifedipine in an amount comprised between 0.1 % and 1 % of the total composition, and optionally, then adding glycerine comprised between 40% and 46% of the total composition. Afterwards, water is added until a total content comprised between 6% and 10% of the total composition, optionally with additional sweeteners or flavouring agents dissolved. The sum total of components is 100%

The liquid solution can be packaged in unitary dose or multidose containers, for example, ampoules, vials, prefilled syringes, cartridges or bags. The final solution may be dosed and administrated by a syringe, dropper, or any suitable quantitative device. Preferably, the solution is dosed by a syringe.

The composition of the present invention has been found to be especially appropriate for several of the off-label indications of nifedipine, for example as a tocolytic agent, i.e. as an inhibitor of preterm labour. The rapid effect onset, low inter-individual variability, and the low toxicological limitations of the composition of the invention render it suitable to administering high daily dosages which are needed for stopping and/or treating acute conditions, for example to stop pre-term contractions ranging from 30 mg/day to 160 mg/day, and dosage regimes of 40-120 mg/day for maintenance treatments lasting for several weeks. The pharmaceutical composition of the invention further can be used for stopping and treating angina, stroke, or Raynaud Syndrome.

Throughout the description and claims the word "comprise" and variations of the word, such as "comprising", is not intended to exclude other technical features, additives, components, or steps. Additional objects, advantages and features of the invention will become apparent to those skilled in the art upon examination of the description or may be learned by practice of the invention. Furthermore, the present invention covers all possible combinations of particular and preferred embodiments described herein. The following examples and drawings are provided by way of illustration, and they are not intended to be limiting of the present invention.

### EXAMPLES

### Example 1: Oral Solution of nifedipine

### Oral solution of nifedipine

| | |
|---|---|
| 0.150 g | Nifedipine |
| 14.000 g | Ethanol 96° |
| 12.600 g | Glycerine |
| 0.150 g | Sodium cyclamate |
| 0.015 g | Sodium saccharine |
| 0.0525 g | Ethylparaben |
| 0.006 g | Colouring agent E-110 |
| 0.02 g | Flavouring Agent |
| q.s. 30.0 ml | Purified water |
| Aprox. 2.4 g | |

### Process for the preparation of an oral solution of nifedipine

Sodium cyclamate and the colouring agent E-110 were dissolved in water. A first alcoholic solution was prepared dissolving ethylparaben, sodium saccharine and the flavouring agent in ethanol 96° (Alcoholic Solution I). Nifedipine were added to the Alcoholic Solution I and the mixture was homogenized. Then, glycerine was added and the solution was mixed. When the mixture became homogeneous, the aqueous solution was added to the alcoholic solution and the mixture was homogenized. Volume was corrected with water and the solution was mixed until obtaining a suitable homogeneous solution.

### Stability

The product has shown to be stable and suitable for administration for at least 12 month at 25°C and 65% of relative humidity, according to the conditions set out for long-term stability studies by the International Conference on Harmonisation of Technical Requirements for the Registration of Pharmaceuticals for Human Use (ICH).

### Example 2: Comparative pharmacokinetic Assay of bioavailability in healthy female volunteers.

Adalat soft-gel capsules 30 mg were tested versus nifedipine ethanol solution of Example 1 in a randomized crossover assay on 36 healthy female volunteers. The aim of the study was to test the bioavailability of the pharmaceutical composition of the invention and the marketed product Adalat^{®} in the pharmaceutical form of soft-gel capsules.

Two treatments, a control treatment comprising the administration of three capsules of Adalat^{®} 10 mg and the test treatment comprising the administration of 6 ml of nifedipine 5 mg/ml solution of Example 1, were administered once to each volunteer with a clearance period of 7 days between each treatment. Volunteers were monitored 24 hours after administration.

Blood samples were extracted before administration, and 10 min, 20 min, 30 min, 45 min, 60 min, 75 min, 90 min, 2 h, 3 h, 5 h, 7 h, 9 h, 12 h, and 24 h from each administration. Blood samples were collected in opaque EDTA K₃ vials and were processed over red light. The vials were centrifuged for 10 min at 3500 rpm at 4°C and two 2.5 ml aliquots of the upper plasmatic phase were stored at -80°C.

Nifedipine plasmatic concentration was quantified by HPLC, according to a validated HPLC method with a 0.5 ng/ml quantification limit.

All kinetic parameters were determined using WinNonlin-Pro 2.1 software from the obtained plasmatic concentration data.

Bioavailability means the rate and extent to which the active substance is absorbed from a pharmaceutical form and becomes available at the systemic circulation. Bioavailability is assessed by serial measurements of the drug in the systemic circulation. These serial measurements provide a plasma concentration-time curve from which some important pharmacokinetic parameters can be calculated, including the area under the curve (AUC), the maximum observed concentration (Cₘₐₓ) and the time when Cₘₐₓ is reached (tₘₐₓ). The AUC provides an estimate of the amount of drug absorbed in the systemic circulation while tₘₐₓ and Cₘₐₓ reflects the rate of absorption.

The present bioavailability test is based in three major pharmacokinetic parameters, the Area Under Curve from time 0 to last experimental time (AUC₀^{t}), Area Under Curve from time 0 to infinite (AUC₀^{∞}) and the maximum plasmatic concentration (Cₘₐₓ).

Major bioavailability parameters were analyzed by one-way ANOVA with two t-tests and 90% confidence limits carried out to assess the conclusions of the study.

Comparative results are shown in Table 1 below.

**Table 1:**

| | Nifedipine Solution Example 2 | | Adalat^{®} Soft-gel capsules | |
|---|---|---|---|---|
| | Arithmetic Mean | SD | Arithmetic Mean | SD |
| AUC₀^{t} (ng.h/ml) | 866.65 | 247.72 (28.6%) | 758.00 | 232.19 (30.6%) |
| AUT₀^{∞} (ng.h/ml) | 887.71 | 259.48 (29.2%) | 778.63 | 235.60 (30.3%) |
| Cmax (ng/ml) | 418.95 | 99.74 (23.8%) | 355.38 | 189.00 (53.2%) |
| | Median | Range (min-max) | Median | Range (min-max) |
| tₘₐₓ (h) | 0.33 | 0.16 - 0.75 | 0.50 | 0.33 - 3.00 |

As extracted from the result data in Table 1, the pharmaceutical composition of the invention showed increased AUC₀^{t}, AUC₀^{∞}, and Cₘₐₓ and decreased tₘₐₓ. Thus, improved bioavailability was assessed for the pharmaceutical compositions of the invention.

### Comparative inter-individual variability of systemic absorption regarding the co-administration of nifedipine and ethanol.

Data obtained from the above comparative study of bioavailability were processed to obtain the inter-individual variability data for Cₘₐₓ. Table 2 shows inter-individual variability data of Cₘₐₓ for the pharmaceutical composition of Example 2, Adalat 30 mg, and the data provided in McGilveray et al., Phar. Res., 1992, vol. 9(5), pgs. 683-686 for the co-administration of Adalat^{®} 20 mg and a 75 ml acute dose of ethanol.

**Table 2:**

| Composition | AUC_{inf} (ng.h/ml) | | | | AUCₜ (ng.h/ml) | | | | Cₘₐₓ (ng/ml) | | | |
|---|---|---|---|---|---|---|---|---|---|---|---|---|
| | Mean | SD | CV | Fᵣₑₗ | Mean | SD | CV | Fᵣₑₗ | Mean | SD | CV | Fᵣₑₗ |
| Adalat 20mg | 360 | 100 | 28% | | 346 | 95 | 27% | | 186 | 126 | 68% | |
| Adalat 20mg + 75 ml EtOH | 552 | 72 | 13% | 153% | 533 | 66 | 12% | 154% | 223 | 99 | 44% | 120% |
| Adalat 30 mg | 779 | 236 | 30% | | 758 | 232 | 31% | | 355 | 189 | 53% | |
| Composition Example 2 30 mg | 888 | 259 | 29% | 114% | 867 | 248 | 29% | 114% | 419 | 100 | 24% | 118% |

The pharmaceutical composition of the invention disclosed in Example 2 comprises 3.47 ml of ethanol, thus the amount of ethanol administered by McGilveray et al. exceeded in about 25-fold that of the pharmaceutical composition of the invention, being unsuitable for therapeutic administration. Surprisingly, the pharmaceutical composition of the invention, even comprising a much reduced amount of ethanol, provides a notable increase of the bioavailability of the corresponding Adalat^{®} composition while reducing the inter-individual variability of Cₘₐₓ.

The results provided by McGilveray et al. show a greater increase of AUC than of Cₘₐₓ (154% vs 120%), what, as is mentioned in their study, suggest an inhibition of the metabolism of nifedipine by the co-administration of an acute dose of alcohol. Thus, the inhibited metabolism of nifedipine leads to a higher amount of the product remaining available systemically.

The pharmaceutical composition of the present invention contains such little amount of ethanol that no significant inhibition of metabolism of nifedipine can be achieved. Further, results obtained by the pharmaceutical composition of the invention show a little difference about the increases of both parameters, but the increase of Cₘₐₓ is slightly greater than of that of AUC (118% vs 114%). These results suggest that the improved bioavailability of the pharmaceutical composition of the invention can be affected, among other factors, by an increase of the systemic absorption instead by the inhibition of the metabolism of the product.

## Claims

1. An oral liquid pharmaceutical composition which is a solution comprising::
a) nifedipine in an amount comprised between 0.1% and 1% w/w;
b) ethanol in an amount comprised between 38% and 58% w/w;
c) water in an amount comprised between 4% and 12% w/w;
d) glycerine in an amount comprised between 30% and 50% w/w; and
e) optionally, other pharmaceutical excipients or carriers;
being the sum total of components 100% w/w,
provided that the composition is substantially free of polyethyleneglycol and of derivatives thereof selected from polyethylene glycol alkyl ethers, polyethylene glycol fatty acid esters, polyethylene glycol sorbitan fatty acid esters and polyethylene glycol oxystearates, wherein substantially free is that the polyethyleneglycol and each of the derivatives thereof are in an amount from 0% to 0.1% w/w.

2. The oral liquid pharmaceutical composition according to claim 1, wherein the amount of glycerine is comprised between 40% and 46% w/w.

3. The oral liquid pharmaceutical composition according to claim 2, wherein the amount of glycerine is comprised between 42% and 44% w/w.

4. The oral liquid pharmaceutical composition according to any of the claims 1-3, wherein the amount of ethanol is comprised between 43% and 53% w/w.

5. The oral liquid pharmaceutical composition according to claim 4, wherein the amount of ethanol is comprised between 45% and 50% w/w.

6. The oral liquid pharmaceutical composition according to any of the claims 1-5, wherein the amount of water is comprised between 6% and 10% w/w.

7. The oral liquid pharmaceutical composition according to claim 6, wherein the amount of water is comprised between 7% and 9% w/w.

8. The oral liquid pharmaceutical composition according to claim 1, which is a solution having the following composition:
a) nifedipine in an amount comprised between 0.3% and 0.7% w/w;
b) ethanol in an amount comprised between 45% and 50% w/w;
c) water in an amount comprised between 7% and 9% w/w; and
d) glycerine in an amount comprised between 42% and 44% w/w.

9. The oral liquid pharmaceutical composition according to claim 8, further comprising:
e) at least one preservative in an amount comprised between 0.05% and 0.5% w/w;
f) at least one sweetener in an amount comprised between 0.02% and 0.8% w/w; and
g) optionally, at least one colouring agent or at least one flavouring agent each of them in an amount comprised between 0.01 % and 0.07% w/w.

10. An oral liquid pharmaceutical composition as defined in any of the claims 1-9, for use in stopping and/or treating an acute condition.

11. An oral liquid pharmaceutical composition according to claim 10, for use in stopping and/or treating preterm labour, angina, stroke, or Raynaud Syndrome.

12. An oral liquid pharmaceutical composition according to claim 11, for use in stopping preterm labour.

## Patentansprüche

1. Orale flüssige pharmazeutische Zusammensetzung, die eine Lösung ist, die folgendes umfasst:
a) Nifedipine in einer Menge, die bei zwischen 0,1 Gew.-% und 1 Gew.-% liegt;
b) Ethanol in einer Menge, die bei zwischen 38 Gew.-% und 58 Gew.-% liegt;
c) Wasser in einer Menge, die bei zwischen 4 Gew.-% und 12 Gew.-% liegt;
d) Glycerin in einer Menge, die bei zwischen 30 Gew.-% und 50 Gew.-% liegt; und
e) wahlweise andere pharmazeutische Hilfsstoffe oder Arzneistoffträger; wobei die gesamte Menge an Bestandteilen 100 Gew.-% ist;
vorausgesetzt, dass die Zusammensetzung im Wesentlichen frei von Polyethylenglycol und/oder Derivaten davon ist, die ausgewählt sind aus Polyethylenglycolalkylethern, Polyethylenglycol-Fettsäureestern, Polyethylenglycolsorbitan-Fettsäureestern und Polyethylenglycol-Oxystearaten, wobei "frei von" im Wesentlichen bedeutet, dass das Polyethylenglycol und jedes der Derivate davon in einer Menge von 0 Gew.-% bis 0,1 Gew.-% vorhanden sind.

2. Orale flüssige pharmazeutische Zusammensetzung nach Anspruch 1, wobei die Menge an Glycerin bei zwischen 40 Gew.-% und 46 Gew.-% liegt.

3. Orale flüssige pharmazeutische Zusammensetzung nach Anspruch 2, wobei die Menge an Glycerin bei zwischen 42 Gew.-% und 44 Gew.-% liegt.

4. Orale flüssige pharmazeutische Zusammensetzung nach einem der Ansprüche 1-3, wobei die Menge an Ethanol bei zwischen 43 Gew.-% und 53 Gew.-% liegt.

5. Orale flüssige pharmazeutische Zusammensetzung nach Anspruch 4, wobei die Menge an Ethanol bei zwischen 45 Gew.-% und 50 Gew.-% liegt.

6. Orale flüssige pharmazeutische Zusammensetzung nach einem der Ansprüche 1-5, wobei die Menge an Wasser bei zwischen 6 Gew.-% und 10 Gew.-% liegt.

7. Orale flüssige pharmazeutische Zusammensetzung nach Anspruch 6, wobei die Menge an Wasser bei zwischen 7 Gew.-% und 9 Gew.-% liegt.

8. Orale flüssige pharmazeutische Zusammensetzung nach Anspruch 1, die eine Lösung der folgenden Zusammensetzung ist:
a) Nifedipin in einer Menge, die bei zwischen 0,3 Gew.-% und 0,7 Gew.-% liegt;
b) Ethanol in einer Menge, die bei zwischen 45 Gew.-% und 50 Gew.-% liegt;
c Wasser in einer Menge, die bei zwischen 7 Gew.-% und 9 Gew.-% liegt; und
d) Glycerin in einer Menge, die bei zwischen 42 Gew.-% und 44 Gew.-% liegt.

9. Orale flüssige pharmazeutische Zusammensetzung nach Anspruch 8, die weiterhin folgendes umfasst:
e) mindestens einen Konservierungsstoff in einer Menge, die bei zwischen 0,05 Gew.-% und 0,5 Gew.-% liegt;
f) mindestens einen Süßstoff in einer Menge, die bei zwischen 0,02 Gew.-% und 0,8 Gew.-% liegt; und
g) wahlweise mindestens einen Farbstoff oder mindestens einen Aromastoff jeweils in einer Menge, die bei zwischen 0,01 Gew.-% und 0,07 Gew.-% liegt.

10. Orale flüssige pharmazeutische Zusammensetzung wie in einem der Ansprüche 1-9 definiert zur Verwendung zum Stoppen und/oder zur Behandlung einer akuten Störung.

11. Orale flüssige pharmazeutische Zusammensetzung nach Anspruch 10 zur Verwendung zum Stoppen und/oder zur Behandlung von vorzeitigen Wehentätigkeit, Angina, Schlaganfall oder Raynaud-Syndrom.

12. Orale flüssige pharmazeutische Zusammensetzung nach Anspruch 11 zum Verwenden um vorzeitige Wehentätigkeit zu stoppen.

## Revendications

1. Composition orale liquide pharmaceutique qui est une solution comprenant:
a) nifédipine dans une quantité comprise entre 0,1 % et 1% du poids total;
b) éthanol dans une quantité comprise entre 38% et 58% du poids total;
c) eau dans une quantité comprise entre 4% et 12% du poids total;
d) glycérine dans une quantité comprise entre 30% et 50% du poids total; et
e) facultativement, des autres excipients ou véhicules pharmaceutiques; étant le total de composants 100% du poids total,
à condition que la composition soit essentiellement livre de polyéthylène glycol et de ses dérivés choisis parmi les ethers alkyliques de polyéthylène glycol, des esters d'acide gras de polyéthylène glycol, des esters d'acide gras de polyéthylène glycol sorbitan et des oxystéarates de polyéthylène glycol, où essentiellement livre veut dire que le polyéthylène glycol et chacun de ses dérivés est présente dans une quantité de 0% à 0,1% du poids total.

2. Composition orale liquide pharmaceutique selon la revendication 1, dans laquelle la quantité de glycérine est comprise entre 40% et 46% du poids total.

3. Composition orale liquide pharmaceutique selon la revendication 2, dans laquelle la quantité de glycérine est comprise entre 42% et 44% du poids total.

4. Composition orale liquide pharmaceutique selon l'une quelconque des revendications 1-3, dans laquelle la quantité d'éthanol est comprise entre 43% et 53% du poids total.

5. Composition orale liquide pharmaceutique selon la revendication 4, dans laquelle la quantité d'éthanol est comprise entre 45% et 50% du poids total.

6. Composition orale liquide pharmaceutique selon l'une quelconque des revendications 1-5, dans laquelle la quantité d'eau est comprise entre 6% et 10% du poids total.

7. Composition orale liquide pharmaceutique selon la revendication 6, dans laquelle la quantité d'eau est comprise entre 7% et 9% du poids total.

8. Composition orale liquide pharmaceutique selon la revendication 1 étant une solution ayant la composition suivante:
a) nifédipine dans une quantité comprise entre 0,3% et 0,7% du poids total;
b) éthanol dans une quantité comprise entre 45% et 50% du poids total:
c) eau dans une quantité comprise entre 7% et 9% du poids total; et
d) glycérine dans une quantité comprise entre 42% et 44% du poids total.

9. Composition orale liquide pharmaceutique selon la revendication 8, comprenant en outre:
e) au moins un agent de conservation dans une quantité comprise entre 0,05% et 0,5% du poids total;
f) au moins un édulcorant dans une quantité comprise entre 0,02% et 0,8% du poids total; et
g) facultativement, au moins un colorant ou au moins un arôme, chacun dans une quantité comprise entre 0,01% et 0,07% du poids total.

10. Composition orale liquide pharmaceutique telle que définie dans l'une quelconque des revendications 1-9 pour l'utilisation pour arrêter et/ou traiter un état aigu.

11. Composition orale liquide pharmaceutique selon la revendication 10 pour l'utilisation pour arrêter et/ou traiter le travail prématuré, l'angine, un accident vasculaire cérébral ou le syndrome de Raynaud.

12. Composition orale liquide pharmaceutique selon la revendication 11 pour l'utilisation pour arrêter le travail prématuré.
